(19) Europäisches Patentamt
European Patent Office
Office européen des brevets

(11) EP 1 875 943 A2

(12) **DEMANDE DE BREVET EUROPEEN**

(43) Date de publication:
**09.01.2008 Bulletin 2008/02**

(51) Int Cl.:
***A61Q 19/08*** *(2006.01)* ***A61K 8/99*** *(2006.01)*
***A61K 8/60*** *(2006.01)*

(21) Numéro de dépôt: **07109641.6**

(22) Date de dépôt: **05.06.2007**

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK YU**

(30) Priorité: **03.07.2006 FR 0652782**

(71) Demandeur: **L'ORÉAL**
**75008 Paris (FR)**

(72) Inventeurs:
- **Pelletier, Pascale**
  **92160 Antony (FR)**
- **Marion, Catherine**
  **92160 Antony (FR)**

(74) Mandataire: **Leonard, Armelle**
**L'OREAL**
**DIPI**
**25-29, Quai Aulagnier**
**92665 Asnières-sur-Seine cedex (FR)**

# (54) Procédé cosmétique pour limiter le creusement du visage dû à l'âge

(57) La présente invention concerne notamment un procédé cosmétique pour diminuer la fonte de la lipostructure de la peau au niveau du visage et/ou du cou, et/ou éviter l'affaissement et/ou le creusement des volumes du visage, par application sur la peau du visage et/ou du cou, d'une composition renfermant, dans un milieu physiologiquement acceptable, au moins un extrait de bactérie filamenteuse non photosynthétique non fructifiante.

En particulier, l'extrait de bactérie est un extrait de *Vitreoscilla filiformis.*

EP 1 875 943 A2

## Description

La présente invention se rapporte à un procédé cosmétique pour diminuer la fonte de la lipo-structure de la peau au niveau du visage et/ou du cou, et/ou éviter l'affaissement et/ou le creusement des volumes du visage, par application sur la peau du visage et/ou du cou, d'une composition renfermant, dans un milieu physiologiquement acceptable, au moins un extrait de bactérie filamenteuse non photosynthétique non fructifiante.

L'invention concerne également une composition cosmétique susceptible d'être utilisée dans le procédé selon l'invention renfermant, dans un milieu physiologiquement acceptable, (i) au moins un extrait de bactérie filamenteuse non photosynthétique non fructifiante et (ii) au moins un dérivé C-glycoside.

Elle porte encore sur l'utilisation cosmétique d'au moins un extrait de bactérie filamenteuse non photosynthétique non fructifiante dans une composition, pour diminuer la perte de densité cutanée et/ou la fonte de la lipo-structure de la peau et/ou éviter l'affaissement et/ou le creusement des volumes du visage,.

Il est connu que les signes du vieillissement cutané ont à la fois des causes chronologiques (vieillissement génétiquement programmé), et des facteurs additionnels aggravants tels que l'exposition au rayonnement UV et surtout les carences hormonales survenant lors de la ménopause. Ils se traduisent par des modifications de l'état de la peau qui sont essentiellement dues à un ralentissement et à un déséquilibre du fonctionnement cutané, qui se traduit par une « atrophie » de l'ensemble des assises de la peau. On observe ainsi une diminution de la qualité du derme (élastine, collagène, glycosaminoglycannes) et une perte de consistance de la matrice extracellulaire ; une diminution de l'épaisseur de l'épiderme (ralentissement du renouvellement cellulaire) ; un ralentissement de la production de lipides et des protéines de structure épidermique; un déséquilibre de la desquamation ; et une réduction du contenu en eau de la peau.

En particulier, et notamment par suite des modifications précitées, on observe que le matelas de soutien de la peau s'affaiblit et la masse adipeuse fond. On parle aussi de fonte de la 'lipo-structure' de la peau.

Par 'lipo-structure de la peau', on entend le réseau de cellules lipidiques qui forme les volumes sur lesquels la peau du visage repose et se moule.

Ainsi le visage se creuse progressivement, la peau perd de sa consistance et de son maintien, la peau a tendance à tomber ; l'ovale s'alourdit ; on assiste à un affaissement et/ou un creusement des volumes du visage.

Ce phénomène est surtout visible au niveau des joues, qui se creusent de plus en plus, du contour de l'oeil, et de l'ovale du visage, qui est moins bien dessiné (apparition de bajoues).

Ces phénomènes s'accentuent avec l'âge et sont particulièrement apparents chez les personnes à peaux matures voire très matures, et notamment chez les femmes ménopausées.

Par 'peaux matures' selon l'invention, on entend notamment des peaux de sujets ayant au moins 40 ans.

Par 'peaux très matures' selon l'invention, on entend notamment des peaux de sujets ayant au moins 50 ans, en particulier au moins 60 ans, voire 65 ans.

Or, on cherche constamment des moyens de contrer, ou tout du moins de retarder, l'apparition de ces signes de vieillissement, et en particulier on cherche de nouveaux moyens permettant de :

- diminuer la perte de densité cutanée et/ou la fonte de la lipo-structure de la peau, en particulier au niveau des joues et du contour de l'oeil, et/ou
- restaurer la lipostructure de la peau, et/ou
- éviter l'affaissement et/ou le creusement des volumes du visage, en particulier au niveau des joues et du contour de l'oeil, et/ou
- augmenter le volume des pommettes du visage, et/ou
- améliorer les volumes qui sous-tendent la peau du visage et/ou du cou, en particulier au niveau des joues, de l'ovale du visage et du contour de l'oeil, et/ou
- améliorer la densité, le rebondi et le maintien de la peau, en particulier au niveau des joues, de l'ovale du visage et du contour de l'oeil, et/ou
- refaçoner les traits du visage, en particulier l'ovale du visage.

La Demanderesse a découvert, de manière surprenante et inattendue, qu'un extrait de bactérie filamenteuse non photosynthétique non fructifiante avait la propriété de stimuler la lipogénèse et favoriser la différenciation adipocytaire, permettant ainsi d'éviter ou de ralentir la fonte des graisses contenues dans les tissus de soutien de la peau, autrement nommée 'fonte de la lipo-structure de la peau'.

C'est dans ce contexte que la Demanderesse a imaginé utiliser un tel extrait de bactérie filamenteuse non photosynthétique non fructifiante pour lutter contre les divers mécanismes de creusement et d'affaissement du visage et/ou du cou.

Les extraits de bactérie filamenteuse non photosynthétique non fructifiante sont déjà connus comme agents immunostimulants (EP 0 604 631), antagonistes de subtance P (EP 0 761 204), stimulant la prolifération des fibroblastes (EP0 681 831), antioxydants (EP 1 354 593), ou encore amincissants (FR 99 00405).

Mais à la connaissance de la Demanderesse, il n'a jamais été décrit ni suggéré l'effet de ces extraits de bactérie filamenteuse non photosynthétique non fructifiante, en particulier de *Vitreoscilla filiformis,* sur la stimulation de la lipogénèse et/ou la différenciation des adipocytes, ni leur utilisaion pour remodeler le visage et/ou le cou et/ou limiter le creusement du visage dû à l'âge.
La présente invention a donc pour objet un procédé cosmétique pour diminuer la fonte de la lipo-structure de la peau au niveau du visage et/ou du cou, et/ou éviter l'affaissement et/ou le creusement des volumes du visage, par application sur la peau du visage et/ou du cou d'une composition renfermant, dans un milieu physiologiquement acceptable, au moins un extrait de bactérie filamenteuse non photosynthétique non fructifiante.
En particulier, le procédé selon l'invention vise à augmenter le volume des pommettes du visage.
Il est notamment destiné à redessiner les contours et/ou l'ovale du visage, et/ou éviter la formation de bajoues et/ou limiter le creusement des joues et/ou du contour de l'oeil.
Selon un mode particulier de l'invention, la composition est appliquée sur les zones de creusement et/ou d'affaissement dû à l'âge du visage et/ou du cou ou sur des personnes présentant un creusement des joues et/ou du contour de l'oeil et/ou un affaissement des joues.
Ces zones de creusement et/ou d'affaissement dû à l'âge sont notamment les joues, le contour de l'oeil et/ou l'ovale du visage (contours du visage).
Les personnes présentant un creusement des joues et/ou du contour de l'oeil et/ou un affaissement des joues peuvent être des hommes et des femmes de tout âge, mais il s'agira en particulier de personnes à peaux matures (ayant au moins 40 ans), voire très matures (ayant au moins 50, voire 60 ans).
Et en particulier, de femmes ménopausées.
Le procédé selon l'invention est donc particulièrement avantageux pour remodeler le visage et/ou le cou, et/ou limiter le creusement du visage des personnes à peaux matures, voire très matures.
Il est également avantageux pour remodeler le visage et/ou le cou, et/ou limiter le creusement du visage des femmes ménopausées.

<u>Extraits de bactérie filamenteuse non photosynthétique non fructifiante</u>

Les extraits de bactéries utilisables selon l'invention sont préparés à partir de bactéries filamenteuses non photosynthétiques telles que définies selon la classification du Bergey's Manual of Systematic Bacteriology (vol. 3, sections 22 et 23, 9˚édition, 1989), parmi lesquelles on peut citer les bactéries appartenant à l'ordre des Beggiatoales, et plus particulièrement les bactéries appartenant aux genres Beggiatoa, Vitreoscilla, Flexithrix ou Leucothrix.
Les bactéries qui viennent d'être définies et dont plusieurs ont déjà été décrites ont généralement un habitat aquatique et peuvent être trouvées notamment dans des eaux marines ou dans des eaux thermales. Parmi les bactéries utilisables, on peut citer par exemple :

*Vitreoscilla filiformis* (ATCC 15551)
*Vitreoscilla beggiatoïdes (A TCC 43181)*
*Beggiatoa alba* (ATCC 33555)
*Flexithrix dorotheae* (ATCC 23163)
*Leucothrix mucor* (ATCC 25107)
*Sphaerotilus natans* (ATCC 13338)

De préférence, on utilisera un extrait de *Vitreoscilla filiformis* (ATCC 15551).
Par 'extrait de bactérie' selon l'invention, on entend un extrait de la biomasse bactérienne ou toute fraction active dudit extrait, en particulier :

(i) des cellules de bactérie isolées du milieu de culture, qui ont été concentrées, par exemple par centrifugation ('extrait cellulaire non stabilisé') ; ou
(ii) des cellules de bactérie concentrées (i), puis soumises à une opération de rupture des enveloppes des cellules de bactérie, par tout moyen connu de l'homme du métier, comme l'action d'ultrasons ou préférentiellement l'autoclavage ('extrait cellulaire stabilisé'). Par 'enveloppes', on entend paroi bactérienne et éventuellement les membranes sous-jacentes ;
(iii) le surnageant obtenu par filtration de l'extrait cellulaire stabilisé (ii),

ou toute fraction active dudit extrait ayant un effet stimulant sur la lipogénèse et/ou la différenciation adipocytaire.
Cette fraction active, dont l'effet sur la lipogénèse et/ou la différenciation adipocytaire peut être évalué selon l'une des méthodes décrites dans les exemples ci-après, peut être obtenue par les méthodes classiques de fractionnement, telles que l'extraction en présence d'un solvant, la précipitation sélective ou l'ultrafiltration tangentielle (UFT)

par exemple.

Ces extraits ou fractions peuvent être conservés par exemple par congélation desdits extraits ou desdites fractions et utilisés après décongélation.

On parlera plus simplement dans le reste de la description 'd'extrait cellulaire' de bactéries ((i) et (ii)), de 'surnageant' dudit extrait (iii) ou de 'fraction active'.

L'extrait de bactérie filamenteuse non photosynthétique non fructifiante utilisable dans la composition utilisée dans le procédé selon l'invention est de préférence choisi parmi un extrait cellulaire, le surnageant dudit extrait cellulaire ou une fraction active dudit extrait cellulaire.

De préférence, l'extrait de bactérie filamenteuse non photosynthétique non fructifiante est un extrait de *Vitreoscilla filiformis,* encore plus préférentiellement un extrait cellulaire de *Vitreoscilla filiformis.*

Pour préparer l'extrait de bactérie selon l'invention, on peut cultiver lesdites bactéries selon les méthodes connues de l'homme du métier, ou se reporter en particulier à la description de la demande de brevet WO-A-94-02158. On obtient un extrait cellulaire dont on peut séparer le surnageant par exemple par filtration et centrifugation. L'extrait peut être utilisé sous forme aqueuse ou sous forme lyophilisée. Le protocole est décrit plus en détail à l'exemple 1 ci-après. Cet extrait de bactérie peut être refractionné et utilisé pur ou dilué à différentes concentrations.

Dans les compositions utilisées dans le procédé selon l'invention, on utilisera généralement de 0,001 à 10%, et en particulier de 0,005 à 2%, en poids d'extrait sec de bactérie filamenteuse non photosynthétique non fructifiante par rapport au poids total de la composition.

On utilisera de préférence une quantité allant de 0,01 à 2% en poids d'extrait sec de bactérie filamenteuse non photosynthétique non fructifiante par rapport au poids total de la composition.

Ces compositions peuvent contenir l'extrait de bactérie filamenteuse non photosynthétique non fructifiante sous forme de dispersion dans un véhicule approprié tel que, par exemple, l'eau, les solvants organiques, les corps gras y compris les huiles, et leurs mélanges, notamment des émulsions.

La composition utilisée dans le procédé selon l'invention est généralement adaptée à une application topique sur la peau et comprend donc généralement un milieu physiologiquement acceptable, c'est-à-dire compatible avec la peau et/ou ses phanères. Il s'agit de préférence d'un milieu cosmétiquement acceptable, c'est-à-dire qui présente une couleur, une odeur et un toucher agréables et ne génère pas d'inconforts inacceptables (picotements, tiraillements, rougeurs), susceptible de détourner la consommatrice d'utiliser cette composition.

La composition selon l'invention peut se présenter sous toutes les formes galéniques classiquement utilisées pour une application topique et notamment sous forme de dispersions du type lotion ou gel aqueux, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème, gel, sérum, ou encore d'émulsions multiples (E/H/E ou H/E/H), de microémulsions, de dispersions vésiculaires de type ionique et/ou non ionique, ou de dispersions cire/phase aqueuse. Ces compositions sont préparées selon les méthodes usuelles.

Selon un mode préféré de réalisation de l'invention, la composition se présente sous forme d'une émulsion huile-dans-eau (H/E).

Il s'agira notamment d'un fluide, d'une crème, d'un gel ou d'un sérum, par exemple d'une crème de jour, d'une crème de nuit, un sérum de contour des yeux.

Comme huiles utilisables dans la composition selon l'invention, on peut citer : les esters d'acides gras et d'alcools gras ; les huiles de silicone, volatiles (telles que les cyclomethicones) ou non volatiles (telles que les dimethicones) ; les alcools gras ramifiés tels que l'octyldodécanol ; les huiles hydrocarbonées telles que la vaseline, le squalane, l'isohexadécane et les huile minérales ; les huiles végétales ; et le beurre de karité. La phase huileuse peut également comprendre des cires telles que la cire d'abeille.

Cette composition peut en outre contenir divers adjuvants couramment utilisés dans le domaine cosmétique, tels que des polyols, en particulier la glycérine, le propylène glycol et les polyéthylène glycols ; des émulsionnants dont les esters d'acides gras et de polyéthylène glycol, les esters d'acides gras et de glycéryle, les esters d'acides gras et de sucrose, les éthers d'alcools gras et de polyéthylène glycol, et les esters d'acides gras et de méthylglucose éventuellement oxyéthylénés ; des co-émulsionnants tels que les alcools cétylique et stéarylique ; des charges, notamment de la silice, et des poudres expansées telles que les microsphères formées d'un terpolymère de chlorure de vinylidène, d'acrylonitrile et de méthacrylate et commercialisées sous la dénomination EXPANCEL par la société Kemanord Plast ; des épaississants et/ou gélifiants tels que les gommes de polysaccharides ou de silicone, les homo- et copolymères d'acrylamide, les homo- et copolymères d'acide acrylique et les homo- et copolymères d'acide acrylamido méthylpropane sulfonique (AMPS) ; des conservateurs ; des séquestrants ; des ajusteurs de pH tels que la triéthanolamine, l'hydroxyde de sodium ou l'acide citrique ; de l'éthanol ; des colorants ; des nacres ; et des parfums.

Bien entendu, l'homme du métier veillera à choisir ce ou ces éventuels composés additionnels et/ou leur quantité de manière telle que les propriétés avantageuses de la composition selon l'invention ne soient pas, ou substantiellement pas, altérées par l'adjonction envisagée.

La composition utilisée dans le procédé selon l'invention permet de ralentir la perte de matière du visage responsable de son creusement et de son affaissement avec l'âge. Pour renforcer les effets de cette composition, celle-ci peut renfermer en outre au moins un agent choisi parmi : les agents favorisant la synthèse de macromolécules dermiques et/ou épidermiques, les agents stimulant la prolifération des kératinocytes, les agents favorisant la synthèse de lipides épidermiques, les agents stimulant le métabolisme cellulaire et les agents favorisant la microcirculation.

Agents favorisant la synthèse de macromolécules dermiques ou épidermiques et/ou prévenant leur dégradation

**[0043]** - les agents augmentant la synthèse des glycosaminoglycannes, tels que : un produit de fermentation du lait par *Lactobacillus vulgaris,* tel que celui commercialisé par la société BROOKS sous la dénomination commerciale Biomin yogourth®; un extrait d'algue brune *Padina pavonica* tel que celui commercialisé par la société ALBAN MÜLLER sous la dénomination commerciale HSP3® ; un extrait de *Saccharomyces cerevisiae* disponible notamment auprès de la société SILAB sous la dénomination commerciale Firmalift® ou auprès de la société LSN sous la dénomination commerciale Cytovitin® ; un extrait de *Laminaria ochroleuca* tel que celui disponible auprès de la société SECMA sous la dénomination commerciale Laminaïne® ; un extrait de *Centella asiatica* tel que celui disponible auprès de la société ROCHE sous la dénomination commerciale ETCA® ; un extrait de cresson (*Nasturtium officinale*) disponible auprès de la société SILAB sous la dénomination commerciale Odraline®; l'essence de Mamaku de Lucas Meyer; le D-xylose, ses esters et les oligosaccharides contenant du D-xylose tels que décrits notamment dans la demande WO99/24009 ; les C-glycosides décrits dans la demande WO 02-051828, tels que le C-ω-D-xylopyranoside-2-hydroxy-propane et ses sels et isomères optiques et géométriques. Un agent augmentant la synthèse des glycosaminoglycannes préféré est le C-ω-D-xylopyranoside-2-hydroxy-propane et ses sels et isomères optiques et géométriques.

**[0044]** - les agents favorisant la synthèse du collagène et/ou prévenant sa dégradation, tels que les extraits de Centella asiatica, les asiaticosides et dérivés ; l'acide ascorbique ou vitamine C et ses dérivés; les peptides de synthèse tels que la iamin, le biopeptide CL ou palmitoyloligopeptide commercialisé par la société SEDERMA ; les peptides extraits de végétaux, tels que l'hydrolysat de soja commercialisé par la société COLETICA sous la dénomination commerciale Phytokine® ou la protéine hydrolysée de soja commercialisée par la société SILAB sous la dénomination Ridulisse® et les hormones végétales telles que les auxines, et les lignanes ; les rétinoïdes et dérivés, l'extrait de malt commercialisé par la société COLETICA sous la dénomination commerciale Collalift® ; les extraits de myrtille ou de romarin ; le lycopène. Un agent préféré favorisant la synthèse du collagène est une protéine hydrolysée de soja commercialisée par la société SILAB sous la dénomination Ridulisse®

**[0045]** - les agents favorisant la synthèse de molécules épidermiques (ex : fillagrine et kératines), tels que l'extrait de lupin commercialisé par la société SILAB sous la dénomination commerciale Structurine® ; l'extrait de bourgeons de hêtre Fagus sylvatica commercialisé par la société GATTEFOSSE sous la dénomination commerciale Gatuline® RC.

Agents favorisant la prolifération des kératinocytes

On peut citer notamment le phloroglucinol ; les extraits de tourteaux de noix commercialisés par la société GATTEFOSSE ; et les extraits de Solanum tuberosum commercialisés par la société SEDERMA ; un extrait de Larrea divaricata tel que le Capislow® de Sederma, les mélanges de papaye, feuilles d'olivier et citron tel que la Xyléine de Vincience, l'extrait de feuille d'hydrangea macrophylla comme l'Amacha liquid E® d'Ichimaru Pharcos, un extrait de levure tel que le Stimoderm® de CLR.

Agents favorisant la synthèse des lipides épidermiques

On peut citer notamment l'acide ascorbique et ses dérivés, et l'acide cinnamique et ses dérivés.

Agents stimulant le métabolisme cellulaire

Comme agents stimulant le métabolisme énergétique des cellules, qui se trouve ralentit lors du vieillissement, on peut citer notamment la biotine, un extrait de Saccharomyces cerevisaie tel que le Phosphovital® de Sederma, le Physiogenyl® de Solabia, un mélange de gluconate de zinc, de cuivre et de magnésium tel que le Sepitonic M3® de Seppic.

Agents favorisant la microcirculation cutanée

On peut citer notamment la caféine, les extraits de ruscus, de marron d'inde, lierre, ginseng, mélilot, le Kombuchka de Sederma, le Pycnogénol, le gluconate de manganèse (Givobio GMn de Seppic), la Visnadine d'Indena, un extrait de lupin (Eclaline de Silab), l'Epaline 100 des Laboratoires carilène, un extrait de fleur de bigarade (Remoduline de ilab), la vitamine P et ses dérivés comme le Permethol de Sochibios, le nicotinate et dérivés, la lysine et dérivés

(comme l'Asparlyne de Solabia). Les agents préférés sont la caféine et les extraits de ruscus et marron d'inde.
Les agents favorisant la microcirculation cutanée seront particulièrement avantageux dans des compositions destinées au contour des yeux.

Ces agents additionnels seront généralement présents dans la composition dans des teneurs allant de 0,001 à 10% en poids par rapport au poids total de la composition, de préférence de 0,01 à 1 % en poids par rapport au poids total de la composition.

En particulier, la composition utilisée dans le procédé selon l'invention comprend (i) au moins un extrait de bactérie filamenteuse non photosynthétique non fructifiante et (ii) au moins un agent augmentant la synthèse des glycosaminoglycannes en tant qu'agent favorisant la synthèse de macromolécules dermiques et/ou épidermiques.

De façon avantageuse, la composition comprendra en outre une association de trois actifs constituée d'un agent augmentant la synthèse de collagène en tant qu'agent favorisant la synthèse de macromolécules dermiques et/ou épidermiques, d'un agent augmentant la synthèse des lipides épidermiques et d'un agent favorisant la prolifération des kératinocytes.

Et selon un mode particulier, en plus de cette association de trois actifs, la composition pourra comprendre au moins un agent stimulant le métabolisme cellulaire et/ou au moins un agent favorisant la microcirculation.

L'agent stimulant le métabolisme cellulaire sera avantageux notamment dans des compositions pour la nuit et l'agent favorisant la microcirculation sera avantageux notamment dans des compositions destinées au contour de l'oeil.

De préférence :

- l'extrait de bactérie filamenteuse non photosynthétique non fructifiante est un extrait de *Vitreoscilla filiformis ;*
- l'agent augmentant la synthèse des glycosaminoglycanes est un dérivé C-glycoside tel que décrit dans la demande WO 02-051828, et en particulier le C-β-D-xylopyranoside-2-hydroxy-propane ou un de ses sels ou isomères optiques et géométriques.
- l'agent favorisant la synthèse collagène est une protéine hydrolysée de soja telle que celle commercialisée par SILAB sous la dénomination RIDULISSE® ;
- l'agent favorisant la synthèse des macromolécules épidermiques est un extrait de bourgeons de hêtre Fagus sylvatica tel que celui commercialisé par la société GATTEFOSSE sous la dénomination commerciale Gatuline® RC.
- l'agent augmentant la prolifération des kératinocytes est le phloroglucinol (1,3,5-trimethoxybenzène) ou l'un de ses dérivés ;
- l'agent favorisant la synthèse des lipides épidermiques est l'acide cinnamique ou l'un de ses dérivés ;
- l'agent stimulant le métabolisme cellulaire est un extrait de *Saccharomyces cerevisiae* tel que le Phosphovital® de Sederma,
- l'agent favorisant la microcirculation cutanée est la caféine.

La composition selon l'invention peut en outre contenir au moins un filtre UVA et/ou UVB. Les filtres solaires peuvent être choisis parmi les filtres organiques, les filtres inorganiques et leurs mélanges.

Les filtres organiques plus particulièrement préférés sont choisis parmi les suivants (cités selon la nomenclature CTFA) : Ethylhexyl Salicylate, Homosalate, Ethylhexyl Methoxycinnamate, Butyl Methoxydibenzoylmethane, Octocrylene, Phenylbenzimidazole Sulfonic Acid, Disodium Phenyl Dibenzimidazole Tetra-sulfonate, Benzophenone-3, Benzophenone-4, Benzophenone-5, 4-Methylbenzylidene camphor, Terephthalylidene Dicamphor Sulfonic Acid, Bis-Ethylhexyloxyphenol Methoxyphenyl Triazine, Ethylhexyl triazone, Diethylhexyl Butamido Triazone, Methylène bis-Benzotriazolyl Tetramethylbutylphénol, Drometrizole Trisiloxane, Polysilicone-15, et parmi les suivants (cités en noms chimiques) : le 2-(4-diethylamino-2-hydroxybenzoyl)-benzoate de n-hexyle, le 1,1-dicarboxy (2,2'-diméthyl-propyl)-4,4-diphénylbutadiène, la 2,4-bis-[5-1 (diméthylpropyl)benzoxazol-2-yl-(4-phenyl)-imino]-6-(2-ethylhexyl)-imino-1,3,5-triazine, et leurs mélanges.

Les filtres inorganiques sont choisis parmi des pigments ou bien encore des nanopigments (taille moyenne des particules primaires : généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium.

L'invention porte également sur une composition cosmétique susceptible d'être utilisée dans le procédé selon l'une quelconque des revendications précédentes renfermant, dans un milieu physiologiquement acceptable, (i) au moins un extrait de bactérie filamenteuse non photosynthétique non fructifiante et (ii) au moins un dérivé C-glycoside.

L'extrait de bactérie filamenteuse non photosynthétique non fructifiante est de préférence un extrait de Vitreoscilla filiformis, notamment un extrait cellulaire de *Vitreoscilla filiformis.*

Les dérivés C-glycosides utilisables dans la composition selon l'invention sont décrits dans la demande WO 02-051828.

Un dérivé C-glycoside convenant à l'invention peut être un composé de formule générale (I) suivante :

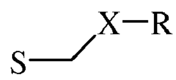
(I)

dans laquelle :

- R représente :

- un radical alkyle linéaire, saturé en $C_1$ à $C_{20}$, en particulier en $C_1$ à $C_{10}$, ou insaturé en $C_2$ à $C_{20}$, en particulier en $C_2$ à $C_{10}$, ou un radical alkyle ramifié ou cyclique, saturé ou insaturé, en $C_3$ à $C_{20}$, en particulier en $C_3$ à $C_{10}$;
- un radical hydrofluoro- ou perfluoro-alkyle, linéaire saturé en $C_1$ à $C_{20}$, en particulier en $C_1$ à $C_{10}$, ou insaturé en $C_2$ à $C_{20}$, en particulier en $C_2$ à $C_{10}$, ou ramifié ou cyclique, saturé ou insaturé, en $C_3$ à $C_{20}$, en particulier en $C_3$ à $C_{10}$;

la chaîne hydrocarbonée constituant lesdits radicaux pouvant, le cas échéant, être interrompue par 1, 2, 3 ou plus d'hétéroatomes choisis parmi :

- un oxygène,
- un soufre,
- un azote, et
- un silicium,

et pouvant être éventuellement substituée par au moins un radical choisi parmi :

- $-OR_4$,
- $-SR_4$,
- $-NR_4R_5$,
- $-COOR_4$,
- $-CONHR_4$,
- -CN,
- un atome d'halogène,
- un radical hydrofluoro- ou perfluoro-alkyle, en $C_1$ à $C_6$,
- un radical cycloalkyle en $C_3$ à $C_8$, et/ou

avec $R_4$ et $R_5$ pouvant représenter, indépendamment l'un de l'autre, un atome d'hydrogène, ou un radical alkyle, perfluoroalkyle ou hydrofluoroalkyle linéaire, saturé en $C_1$ à $C_{30}$, notamment en $C_1$ à $C_{12}$, ou insaturé en $C_2$ à $C_{30}$, notamment en $C_2$ à $C_{12}$, ou ramifié ou cyclique, saturé ou insaturé, en $C_3$ à $C_{30}$, notamment en $C_3$ à $C_{12}$; ou un radical aryle en $C_6$ à $C_{10}$,

- X représente un radical choisi parmi les groupements :

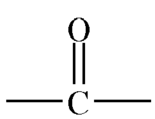

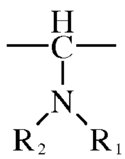

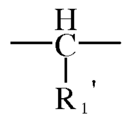

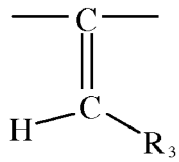

avec $R_1$, $R_2$ et $R_3$ représentant, indépendamment l'un de l'autre, un atome d'hydrogène,

ou un radical R, avec R tel que défini précédemment, et $R'_1$ représente un atome d'hydrogène, un groupe -OH ou un radical R tel que défini précédemment, $R_1$ pouvant désigner également un radical aryle en $C_6$ à $C_{10}$;

- S représente un monosaccharide ou un polysaccharide comportant jusqu'à 20 unités sucre, en particulier jusqu'à 6 unités sucre, sous forme pyranose et/ou furanose et de série L et/ou D, ledit mono- ou poly-saccharide pouvant être substitué par un groupement hydroxyle obligatoirement libre, et éventuellement une ou plusieurs fonction(s) amine(s) éventuellement protégée(s), et
- la liaison S-$CH_2$-X représente une liaison de nature C-anomérique, qui peut être $\alpha$ ou $\beta$,

ainsi que leurs sels cosmétiquement acceptables, leurs solvates tels que les hydrates et leurs isomères.

Dans le cadre de la présente invention, par « halogène », on entend le chlore, le fluor, le brome ou l'iode.

Le terme « aryle »désigne un cycle aromatique tel que phényle, éventuellement substitué par un ou plusieurs radicaux alkyles en $C_1$-$C_4$.

Le terme « cycloalkyle en $C_3$ à $C_8$ »désigne un cycle aliphatique ayant de 3 à 8 atomes de carbone, incluant par exemple le cyclopropyle, le cyclopentyle et le cyclohéxyle.

Parmi les groupes alkyle convenant à la mise en oeuvre de l'invention, on peut notamment citer les groupes méthyle, éthyle, isopropyle, n-propyle, n-butyle, t-butyle, isobutyle, sec-butyle, pentyle, n-hexyle, cyclopropyle, cyclopentyle, cyclohexyle, et allyle.

Selon un mode de réalisation de l'invention, on peut utiliser un dérivé C-glycoside répondant à la formule (I) pour lequel S peut représenter un monosaccharide ou un polysaccharide contenant jusqu'à 6 unités sucre, sous forme pyranose et/ou furanose et de série L et/ou D, ledit mono- ou polysaccharide présentant au moins une fonction hydroxyle obligatoirement libre et/ou éventuellement une ou plusieurs fonctions amine obligatoirement protégée, X et R conservant par ailleurs l'ensemble des définitions précédemment données.

Avantageusement, un monosaccharide de l'invention peut être choisi parmi le D-glucose, le D-galactose, le D-mannose, le D-xylose, le D-lyxose, le L-fucose, le L-arabinose, le L-rhamnose, l'acide D-glucuronique, l'acide D-galacturonique, l'acide D-iduronique, la N-acétyl-D-glucosamine, la N-acétyl-D-galactosamine et désigne avantageusement le D-glucose, le D-xylose, la N-acétyl-D-glucosamine ou le L-fucose, et en particulier le D-xylose.

Plus particulièrement, un polysaccharide de l'invention contenant jusqu'à 6 unités sucre peut être choisi parmi le D-maltose, le D-lactose, le D-cellobiose, le D-maltotriose, un disaccharide associant un acide uronique choisi parmi l'acide D-iduronique ou l'acide D-glucuronique avec une hexosamine choisie parmi la D-galactosamine, la D-glucosamine, la N-acétyl-D-galactosamine, la N-acétyl-D-glucosamine, un oligosaccharide contenant au moins un xylose qui peut être avantageusement choisi parmi le xylobiose, le méthyl-$\beta$-xylobioside, le xylotriose, le xylotétraose, le xylopentaose et le xylohexaose et notamment le xylobiose qui est composé de deux molécules de xylose liées par une liaison 1-4.

Plus particulièrement, S peut représenter un monosaccharide choisi parmi le D-glucose, le D-xylose, le L-fucose, le D-galactose, le D-maltose et notamment le D-xylose.

Selon un autre mode de réalisation de l'invention, on peut utiliser des dérivés C-glycoside répondant à la formule (I) pour lesquels X représente un groupement choisi parmi -CO-, -CH(OH)-, -$CH(NR_1R_2)$-, -CH(R)-, en particulier -CO-, -CH(OH)-, -$CH(NH_2)$-, -$CH(NHCH_2CH_2CH_2OH)$-, -CH(NHPh)-, -$CH(CH_3)$-, et plus particulièrement un groupement -CO-, -CH(OH)-, -$CH(NH_2)$-, et préférentiellement un groupement - CH(OH)-, S et R conservant par ailleurs l'ensemble des définitions précédemment données.

Selon un autre mode de réalisation de l'invention, on peut utiliser un dérivé C-glycoside répondant à la formule

(I) pour lesquels R représente un radical alkyle en linéaire, saturé en $C_1$ à $C_{20}$, en particulier en $C_1$ à $C_{10}$, ou insaturé en $C_2$ à $C_{20}$, en particulier en $C_2$ à $C_{10}$, ou un radical alkyle ramifié ou cyclique, saturé ou insaturé, en $C_3$ à $C_{20}$; en particulier en $C_3$ à $C_{10}$, et éventuellement substitué comme décrit précédemment, S et R conservant par ailleurs l'ensemble des définitions précédemment données. De préférence, R désigne un radical linéaire en $C_1$-$C_4$, notamment $C_1$-$C_3$, éventuellement substitué par -OH, -COOH ou -COOR"$_2$, R"$_2$ étant un radical alkyle saturé en $C_1$-$C_4$, notamment éthyle. Préférentiellement R désigne un radical alkyle linéaire non substitué en $C_1$-$C_4$, notamment $C_1$-$C_2$, en particulier éthyle.

Parmi les dérivés C-glycoside de formule (I), on utilise de préférence ceux pour lesquels :

- R représente un radical alkyle en linéaire, saturé en $C_1$ à $C_{20}$, en particulier en $C_1$ à $C_{10}$, ou insaturé en $C_2$ à $C_{20}$, en particulier en $C_2$ à $C_{10}$, ou un radical alkyle ramifié ou cyclique, saturé ou insaturé, en $C_3$ à $C_{20;}$ en particulier en $C_3$ à $C_{10}$, et éventuellement substitué comme décrit précédemment ;
- S représente un monosaccharide comme décrit précédemment ;
- X représente -CO-, -CH(OH)-, -CH($NR_1R_2$)-, -CH(R)- comme décrit précédemment.

De préférence, on utilise un dérivé C-glycoside de formule (I) pour lesquels :

- R désigne un radical linéaire en $C_1$-$C_4$, notamment $C_1$-$C_3$, éventuellement substitué par -OH, -COOH ou -COOR"$_2$, R"$_2$ étant un radical alkyle saturé en $C_1$-$C_4$, notamment éthyle ;
- S représente un monosaccharide comme décrit précédemment ;
- X représente un groupement choisi parmi -CO-, -CH(OH)-, -CH($NH_2$)-,
- CH($NHCH_2CH_2CH_2OH$)-, -CH(NHPh)-, -CH($CH_3$)-, et plus particulièrement un groupement -CO-, -CH(OH)-, -CH ($NH_2$)-,et préférentiellement un groupement -CH(OH)-.

Préférentiellement, on utilise un dérivé C-glycoside de formule (I) pour lesquels :

- R désigne un radical alkyle linéaire non substitué en $C_1$-$C_4$, notamment $C_1$-$C_2$, en particulier éthyle ;
- S représente un monosaccharide comme décrit précédemment; notamment le D-glucose, le D-xylose, la N-acétyl-D-glucosamine ou le L-fucose, et en particulier le D-xylose ;
- X représente un groupement choisi parmi -CO-, -CH(OH)-, -CH($NH_2$)-, et préférentiellement un groupement -CH (OH)-.

Les sels acceptables pour l'usage non thérapeutique des composés décrits dans la présente invention comprennent des sels non toxiques conventionnels desdits composés tels que ceux formés à partir d'acides organiques ou inorganiques. A titre d'exemple, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Lorsque le composé de formule (I) comporte un groupe acide, la neutralisation du ou des groupes acides peut être effectuée par une base minérale, telle que LiOH, NaOH, KOH, $Ca(OH)_2$, $NH_4OH$, $Mg(OH)_2$ ou $Zn(OH)_2$; ou par une base organique telle qu'une alkylamine primaire, secondaire ou tertiaire, par exemple la triéthylamine ou la butylamine. Cette alkylamine primaire, secondaire ou tertiaire peut comporter un ou plusieurs atomes d'azote et/ou d'oxygène et peut donc comporter par exemple une ou plusieurs fonctions alcool; on peut notamment citer l'amino-2-méthyl-2-propanol, la triéthanolamine, la diméthylamino-2-propanol, le 2-amino-2-(hydroxyméthyl)-1,3-propanediol. On peut encore citer la lysine ou la 3-(diméthylamino)propylamine.

Les solvates acceptables pour les composés décrits dans la présente invention comprennent des solvates conventionnels tels que ceux formés lors de la dernière étape de préparation desdits composés du fait de la présence de solvants. A titre d'exemple, on peut citer les solvates dus à la présence d'eau ou d'alcools linéaires ou ramifiés comme l'éthanol ou l'isopropanol.

A titre illustratif et non limitatif des dérivés C-glycoside convenant plus particulièrement à l'invention, on peut notamment citer les dérivés suivants :

- le C-β-D-xylopyranoside-n-propane-2-one,
- le C-α- D-xylopyranoside-n-propane-2-one,
- le C-β-D-xylopyranoside-2-hydroxy-propane,
- le C-α- D-xylopyranoside-2-hydroxy-propane,

- la 1-(C-β-D-fucopyranoside)-propane-2-one,
- la 1-(C-α-D-fucopyranoside)-propane-2-one,
- la 1-(C-β-L-fucopyranoside)-propane-2-one,
- la 1-(C-α-L-fucopyranoside)-propane-2-one,
- le 1-(C-β-D-fucopyranoside)-2-hydroxy-propane,
- le 1-(C-α-D-fucopyranoside)-2-hydroxy-propane,
- le 1-(C-β-L-fucopyranoside)-2-hydroxy-propane,
- le 1-(C-α-L-fucopyranoside) -2-hydroxy-propane,
- le 1-(C-β-D-glucopyranosyl)-2-hydroxyl-propane,
- le 1-(C-α-D-glucopyranosyl)-2-hydroxyl-propane,
- le 1-(C-β-D-galactopyranosyl)-2-hydroxyl-propane,
- le 1-(C-α-D-galactopyranosyl)-2-hydroxyl-propane
- la 1-(C-β-D-fucofuranosyl)-propane-2-one,
- la 1-(C-α-D-fucofuranosyl)-propane-2-one
- la 1-(C-β-L-fucofuranosyl)-propane-2-one,
- la 1-(C-α-L-fucofuranosyl)-propane-2-one,
- le C-β-D-maltopyranoside-n-propane-2-one,
- le C-α-D-maltopyranoside-n-propane-2-one
- le C-β-D-maltopyranoside-2-hydroxy-propane,
- le C-α-D-maltopyranoside-2-hydroxy-propane, leurs isomères et leurs mélanges.

Selon un mode de réalisation, le C-β-D-xylopyranoside-2-hydroxy-propane ou le C-α-D-xylopyranoside-2-hydroxy-propane, et mieux le C-β-D-xylopyranoside-2-hydroxy-propane, peuvent être avantageusement mis en oeuvre pour la préparation d'une composition selon l'invention.

Selon un mode de réalisation particulier, le dérivé C-glycoside est le C-α-D-xylopyranoside-2-hydroxy-propane sous forme d'une solution à 30 % en poids en matière active dans un mélange eau/propylène glycol (60/40 % en poids) tel que le produit fabriqué par CHIMEX sous la dénomination commerciale « MEXORYL SBB® ».

Bien entendu, selon l'invention, un dérivé C-glycoside répondant à la formule (I) peut être utilisé seul ou en mélange avec d'autres dérivés C-glycosides et en toute proportion.

Un dérivé C-glycoside convenant à l'invention peut notamment être obtenu par la méthode de synthèse décrite dans le document WO 02/051828.

La quantité de dérivé C-glycoside à mettre en oeuvre dans une composition selon l'invention dépend de l'effet cosmétique ou thérapeutique recherché, et peut donc varier dans une large mesure.

L'homme de l'art peut aisément, sur la base de ses connaissances générales, déterminer les quantités appropriées.

Une composition selon l'invention peut comprendre un dérivé C-glycoside à raison d'environ 0,0001 % à environ 25 % en poids en matière active par rapport au poids total de la composition, et en particulier d'environ 0,001 % à environ 10 % en poids en matière active, et plus particulièrement d'environ 0,05 % à environ 5 % en poids en matière active de dérivé C-glycoside par rapport au poids total de la composition.

L'invention porte également sur une composition cosmétique susceptible d'être utilisée dans le procédé selon l'une quelconque des revendications précédentes renfermant, dans un milieu physiologiquement acceptable, (i) au moins un extrait de bactérie filamenteuse non photosynthétique non fructifiante et (ii) au moins un agent favorisant la synthèse des glycosaminoglycanes et (iii) au moins une association de trois actifs constituée d'un agent augmentant la synthèse de collagène, d'un agent augmentant la synthèse des lipides épidermiques et d'un agent favorisant la prolifération des kératinocytes.
Des exemples de ces agents pour chaque classe sont décrits précédemment dans la description.

Une composition préférée selon l'invention comprend au moins un extrait de *Vitreoscilla filiformis,* un C-β-D-xylopyranoside-2-hydroxy-propane ou un de ses sels ou isomères optiques et géométriques, une protéine hydrolysée de soja, un acide cinnamique ou l'un de ses dérivés et un phloroglucinol ou l'un de ses dérivés.

L'invention porte encore sur l'utilisation cosmétique d'au moins un extrait de bactérie filamenteuse non photosynthétique non fructifiante dans une composition, pour diminuer la perte de densité cutanée et/ou la fonte de la lipostructure de la peau et/ou éviter l'affaissement et/ou le creusement des volumes du visage, la perte de consistance de la peau et/ou son maintien.

Elle concerne également l'utilisation cosmétique d'au moins un extrait de bactérie filamenteuse non photosynthétique non fructifiante dans une composition, pour redessiner les contours et/ou l'ovale du visage, et/ou éviter la formation de bajoues et/ou limiter le creusement des joues et/ou du contour de l'oeil.

L'invention sera maintenant illustrée par les exemples non limitatifs suivants. Dans ces exemples, les quantités sont indiquées en pourcentage pondéral.

EXEMPLES

Exemple 1 - Préparation d'un extrait de *Vitreoscilla filiformis*

La souche de *Vitreoscilla filiformis* (ATCC 15551) est mise en culture selon le procédé décrit dans la demande de brevet WO-A-94-02158.
Il s'agit d'un procédé de culture en continu. La culture s'effectue à 26°C durant au moins 48 heures jusqu'à l'obtention d'une concentration cellulaire convenable correspondant à une densité optique à 600 nm supérieure ou égale à 1,5. On repique la souche à 2 % V/V dans du milieu neuf durant 48 heures environ jusqu'à l'obtention d'une culture stable. Un Erlenmeyer de 1 litre contenant 200 ml de milieu neuf est alors ensemencé avec 4 ml de la culture précédente. La culture en Erlenmeyer s'effectue à 26°C sur une table de culture agitée à 100 tours/minutes. Le pied de cuve ainsi obtenu sert d'inoculum à un fermenteur de 50 litres. La croissance s'effectue à 26°C, pH 7, 100 tours/minute et $pO_2 \geq 15$ %. Après 30 heures de croissance, la biomasse est transférée dans un fermenteur de 3000 litres utiles, pour être cultivée dans les mêmes conditions. Après 48 heures de croissance on récolte les cellules en continu. La biomasse est alors concentrée 50 fois environ par centrifugation. Les cellules obtenues sont alors congelées au fur et à mesure de la culture continue. Ces cellules peuvent être utilisées en l'état après décongélation (extrait cellulaire non stabilisé) ou bien être stabilisées par autoclavage à 121°C durant 20 à 40 minutes (extrait cellulaire stabilisé). Les cellules ont alors éclaté durant la stérilisation en libérant le cytosol et en agglomérant les protéines ainsi que les parois. Le produit obtenu est alors biphasique.
La phase liquide surnageante peut être filtrée à 0,22 μm pour éliminer les particules ('surnageant').
L'extrait de bactérie, sous forme d'extrait cellulaire (stabilisé ou non) ou de surnageant, est utilisable en l'état (forme aqueuse) ou peut être lyophilisé suivant les techniques classiques (forme lyophilisée).

Exemple 2 : Mise en évidence des propriétés d'un extrait de *Vitreoscilla filiformis* sur la lipogénèse et la différenciation des adipocytes

L'activité d'un extrait cellulaire de *Vitreoscilla filiformis* tel que préparé selon l'exemple 1 sur un développement local adipeux a été évaluée. L'étude a été réalisée sur des adipocytes humains issus de la zone abdominale. Différents paramètres représentant la physiologie adipocytaire ont été analysés :

a) stimulation de la lipogénèse par mesure de l'incorporation de $^{14}C$-acétate dans les lipides ;
b) stimulation de la différenciation adipocytaire par mesure de l'expression de marqueurs de différenciation adipocytaire : la LPL (lipoprotéine lipase, impliquée dans le stockage des graisses ;
c) suivi de l'enrichissement en lipides par coloration spécifique (AdipoRed™) et imagerie ;
d) suivi de la taille/granulométrie cellulaire par cytométrie de flux et marquage fluorescent AdipoRed™).

L'extrait de *Vitreoscilla filiformis* préparé selon l'exemple 1 a été testé à :

- 10%, 1 % et 0,1 % pour l'effet sur la lipogénèse ;
- 0.25% et 0.05% pour l'effet sur la différenciation adipocytaire.

Stimulation de la lipogénèse par mesure de l'incorporation de $^{14}C$-acétate dans les lipides

Les adipocytes ont été isolés à partir d'une plastie abdominale puis incubés en présence de collagènase ensuite lavés et repris avec du milieu d'essai. Ce milieu d'essai comportait :

- Produit à tester (extrait de *Vitreoscilla filiformis* aux différentes concentrations/Théophylline 1 mM/ Isoprotérénol 1μM), ou rien pour le témoin
- bicarbonate 1.87 mg/ml
- pénicilline / streptomycine 25UI/ml/25μg/ml
- glutamine 2mM
- MEM sans rouge phénol qsp100% (v/v) additionné d'albumine bovine sérique délipidée à 0.5 % (p/v).

Les produits à tester et les adipocytes ont été pré-incubés à 37°C pendant 1h, puis on a ajouté du $^{14}C$-acétate à 50μCi/ml. Après 4h de traitement, les lipides ont été extraits au méthanol/chloroforme/eau et la radioactivité incorporée a été comptée en scintillation liquide.
Les résultats sont présentés dans le tableau suivant :

| Traitement | | % témoin | p | % quenching |
|---|---|---|---|---|
| témoin | - | 100 | - | 0% |
| cérulénine | 20μM | 34 | <0,01 | 0% |
| isoprotérénol | 1μM | 3400 | <0,01 | 0% |
| Extrait de *Vitreoscilla filiformis* | 10%<br>1%<br>0,1% | 585<br>643<br>543 | <0,01<br><0,01<br><0,01 | 66%<br>0%<br>0% |

La cérulénine (inhibiteur d'acide gras synthéthase, FAS) testé à 0,02mM a inhibé de façon significative l'incorporation d'acétate dans les lipides, ce qui valide le test.

L'extrait de *Vitreoscilla filiformis* a stimulé l'incorporation d'acétate de façon significative pour les 3 concentrations testées, mais à la plus forte concentration, le produit a interféré avec le dosage (quenching).

Stimulation de la différenciation adipocytaire par mesure de l'expression d'un marqueur de différenciation adipocytaire : le LPL ou lipoproteine lipase, impliquée dans le stockage des graisses

Les pré-adipocytes humains normaux ont été cultivés en milieu de croissance PGM à 37°C et 5% $CO_2$ jusqu'à confluence. A confluence (J0), les cellules ont été placées en milieu de différenciation (série 1, cellules différenciées) ou milieu de croissance normal (série 2, cellules non différenciées), contenant ou non (témoin) les produits à l'essai ou la référence (TNFα, inhibiteur de différenciation). Les cellules ont ensuite été incubées à 37°C et 5% $CO_2$ pendant 4 jours.

A la fin du traitement, les surnageants ont été éliminés et les cellules rincées en PBS et un volume de 300μL de Tri-Reagent a été ajouté dans chaque puits. L'expression du marqueur LPL a été évaluée par RT-Q-PCR sur les ARN messagers extraits des tapis cellulaires de chaque traitement. On a utilisé le système Light Cycler (Roche Molecular Systems Inc) selon les recommandations du fournisseur. Les couples de primers qui ont été utilisés permettent d'amplifier les fragments spécifiques suivants:

- human β-actin (n° GenBank X00351) comme référence;
- lipoprotein lipase precursor LPL (N° GenBank M15856).

L'incorporation de fluorescence dans l'ADN amplifié est mesurée en continu au cours des cycles de PCR. On évalue une valeur d'expression relative pour le marqueur LPL, exprimée en unités arbitraires selon la formule suivante :

$$(1/2^{\text{nombre de cycles}}) \times 10^6$$

Les résultats sont exprimés en % d'expression relative par rapport au témoin.

| Traitement | | LPL |
|---|---|---|
| Contrôle cellules non différenciées | - | 1 |
| Témoin cellules différenciées | - | 100 |
| TNFα | 25ng/ml | 111 |
| Extrait de *Vitreoscilla filiformis* | 0,25%<br>0,05% | 17224<br>16947 |

Le marqueur LPL est stimulé par l'extrait de *Vitreoscilla filiformis ;* cela traduit un effet positif sur la lipogénèse, ce qui confirme l'effet observé dans le test précédent.

Suivi de l'enrichissement en lipides par coloration spécifique (AdipoRed™) et imagerie

A la fin des traitements, les cellules ont été rincées en PBS puis les lipides intracellulaires ont été colorés par

l'AdipoRed de BioWhittaker. Les cellules colorées ont été observées par microscopie optique, au lecteur Spectramax. Les résultats sont exprimés en % d'augmentation par rapport au témoin.

Mesure sur cellules différenciées

| traitement | | % témoin | p |
|---|---|---|---|
| Témoin (cellules différenciées) | - | 100 | - |
| Contrôle cellules non différenciées | - | 65 | <0,01 |
| TNFα | 25ng/ml | 83 | >0,05 |
| Extrait de *Vitreoscilla filiformis* | 0,25%<br>0,05% | 360<br>150 | <0,01<br><0,01 |

Après 4 jours de culture en milieu de différenciation, les adipocytes présentent un taux de lipides intracellulaires augmenté d'un facteur 1,5 par rapport au milieu de croissance. Le TNFα diminue le taux de lipides comme attendu. L'extrait de *Vitreoscilla filiformis* a induit une augmentation dose-dépendante significative du taux de lipides dans les préadipocytes.

La même expérience réalisée sur des cellules non différenciées a induit également une augmentation significative du taux de lipides dans les préadipocytes, respectivement 564% par rapport au témoin pour la concentration de 0,25% d'extrait de bactérie et 196% par rapport au témoin pour la concentration de 0,05% d'extrait de bactérie.

Suivi de la taille/granulométrie cellulaire par cytométrie de flux et marquage fluorescent AdipoRed™.

A la fin des traitements, les cellules ont été rincées en PBS puis les lipides intracellulaires ont été colorés par l'AdipoRed de BioWhittaker.

A la fin de l'incubation, les cellules ont été rincées, trypsinisées puis analysées par cytométrie de flux. La différenciation des pré-adipocytes se traduit au niveau morphologique par une augmentation du nombre de vésicules lipidiques intracytoplasmiques et une diminution de la taille des cellules.

Les résultats sont exprimés en % d'augmentation par rapport au témoin.

Mesure AdipoRed sur les cellules différenciées

| Traitement | | % témoin | p |
|---|---|---|---|
| Témoin (cellules différenciées) | - | 100 | - |
| Contrôle cellules non différenciées | - | 57 | <0,01 |
| TNFα | 25ng/ml | 80 | <0,01 |
| Extrait de *Vitreoscilla filiformis* | 0,25%<br>0,05% | 115<br>111 | <0,01<br><0,05 |

Les résultats obtenus par ce test confirment ceux précédemment obtenus par la mesure au lecteur Spectramax.

La même expérience réalisée sur des cellules non différenciées induit également une augmentation significative du taux de lipides dans les préadipocytes, respectivement 131% par rapport au témoin pour la concentration de 0,25% d'extrait de bactérie et 124% par rapport au témoin pour la concentration de 0,05% d'extrait de bactérie.

L'ensemble de ces tests montre que l'extrait de bactérie filamenteuse non photosynthétique non fructifiante a un effet sur la lipogénèse et la différenciation des préadipocytes en adipocytes. Cet effet est mis à profit dans les compositions de l'invention pour prévenir ou diminuer le creusement du visage dû à l'âge, en particulier au niveau des joues et du contour de l'oeil.

Exemple 3 : Formulations

1) Crème de soin anti-creusement visage et cou

| | | |
|---|---|---|
| Extrait de Vitreoscilla filiformis selon l'exemple 1 C-β-D-xylopyranoside-2-hydroxy-propane à 30 % en poids en matière active (MA) dans un mélange | | 0,05% |
| eau/1,2propanediol 60/40 | | 1,0% en MA |
| 1,3,5 trimethoxybenzène (phloroglucinol) | | 0,01 % |
| Acide trans-cinnamique | | 0,01% |
| Extrait de protéine de soja (Ridulisse® de SILAB) | | 0,5% |
| Huiles | | 12% |
| Alcool stéarylique | | 1,1% |
| Tensioactifs | | 9,0% |
| Emulsionnants | | 8,0% |
| Conservateurs | | 0,75% |
| Parfums | | 0,4% |
| Eau | qsp | 100% |

La crème de soin anti-creusement visage et cou est appliquée quotidiennement sur la peau du visage et du cou, en particulier au niveau des joues et du cou pour éviter le creusement et/ou l'affaissement de la peau au niveau des joues et du cou.

<u>2) Soin de contour des yeux</u>

| | | |
|---|---|---|
| Caféine | | 0,2% |
| Extrait de *Vitreoscilla filiformis* selon l'exemple 1 | | 0,1% |
| 1,3,5 trimethoxybenzène | | 0,015% |
| Acide trans-cinnamique | | 0,015% |
| Extrait de protéine de soja | | 1,0% |
| Silice | | 5,0% |
| Huiles | | 4,0% |
| Alcools gras | | 3,0% |
| Acide stéarique | | 3,0% |
| Glycérine | | 7,0% |
| Cyclohexasiloxane | | 6,0% |
| Surfactants | | 3,0% |
| Gélifiants | | 1,5% |
| Conservateurs | | 0,9% |
| Eau | qsp | 100% |

Le soin de contour des yeux est appliqué quotidiennement sur le contour de l'oeil, pour éviter le creusement au niveau du contour des yeux.

3) Crème de soin de la peau (émulsion H/E)

| | |
|---|---|
| Glycérine | 5% |
| Disodium EDTA | 0,05% |
| Tensioactifs | 4,0% |
| Conservateurs | 0,85% |
| Caprylyl glycol | 0,5% |
| Alcool stéarylique | 1,1% |
| Acide stéarique | 3,3% |

(suite)

| | | |
|---|---|---|
| Cire d'abeille | | 1% |
| Huiles | | 21% |
| Extrait de Vitreoscilla filiformis selon l'exemple 1 | | 0,025% |
| Extrait de protéine de soja (Ridulisse® de SILAB) | | 0,03% |
| Amidon de maïs | | 3% |
| Hydroxypropyl Tetrahydropyrantriol | | 0,35% |
| Parfums | | 0,4% |
| Acrylamide/Sodium Acryloyldimethyltaurate Copolymer /Isohexadecane / Polysorbate 80 (Simulgel 600) | | 1,26% |
| Eau | qsp | 100% |

Cette crème de soin a fait l'objet d'un test cosmétoclinique décrit ci-dessous : Protocole :

Nombre de sujets : 38

Critères d'inclusion : Femmes âgés de moins de 65 ans (âge moyen = 58 ans) ménopausées depuis au moins 3 ans, sans THS (traitement hormonal substitutif) depuis au moins 6 mois, tous types de peau, ayant des problèmes de fermeté au niveau du visage
Durée du traitement : 4 semaines - 2 fois par jour
Méthode d'évaluation : projection de franges
Schéma expérimental : randomise

Résultats :

Dans les conditions de l'essai, les mesures par projection de franges ont mis en évidence une augmentation significative du volume de la pommette.

**Revendications**

1. Procédé cosmétique pour diminuer la fonte de la lipo-structure de la peau au niveau du visage et/ou du cou et/ou éviter l'affaissement et/ou le creusement des volumes du visage, par application sur la peau d'une composition renfermant, dans un milieu physiologiquement acceptable, au moins un extrait de bactérie filamenteuse non photosynthétique non fructifiante.

2. Procédé selon la revendication 1, **caractérisé en ce qu'**il vise à augmenter le volume des pommettes du visage.

3. Procédé selon la revendication 1 ou 2, **caractérisé en ce que** la composition est appliquée sur les joues, sur le contour de l'oeil et/ou sur les contours du visage.

4. Procédé cosmétique selon l'une quelconque des revendications précédentes, **caractérisé en ce qu'**il est destiné à remodeler le visage et/ou le cou et/ou limiter le creusement du visage des personnes à peaux matures, voire très matures.

5. Procédé cosmétique selon l'une quelconque des revendications précédentes, **caractérisé en ce qu'**il est destiné à remodeler le visage et/ou le cou et/ou limiter le creusement du visage des femmes ménopausées.

6. Procédé cosmétique selon l'une quelconque des revendications précédentes, **caractérisé en ce que** l'extrait de bactérie filamenteuse non photosynthétique non fructifiante est choisi parmi un extrait cellulaire, le surnageant dudit extrait cellulaire ou une fraction active dudit extrait cellulaire.

7. Procédé cosmétique selon l'une quelconque des revendications précédentes, **caractérisé en ce que** l'extrait de bactérie filamenteuse non photosynthétique non fructifiante est un extrait de *Vitreoscilla filiformis.*

8. Procédé selon l'une quelconque des revendications précédentes, **caractérisé en ce que** l'extrait de bactérie filamenteuse non photosynthétique non fructifiante est présent dans la composition en une quantité allant 0,001 à 10% en poids d'extrait sec de bactérie par rapport au poids total de la composition, de préférence de 0,005 à 2% en poids d'extrait sec de bactérie par rapport au poids total de la composition.

**9.** Procédé selon l'une quelconque des revendications précédentes, **caractérisé en ce que** la composition comprend en outre au moins un agent additionnel choisi parmi : les agents favorisant la synthèse de macromolécules dermiques et/ou épidermiques, les agents stimulant la prolifération des kératinocytes, les agents favorisant la synthèse de lipides épidermiques, les agents stimulant le métabolisme cellulaire et les agents favorisant la microcirculation.

**10.** Procédé selon la revendication 9, **caractérisé en ce que** la composition comprend (i) au moins un extrait de bactérie filamenteuse non photosynthétique non fructifiante et (ii) au moins un agent augmentant la synthèse des glycosaminoglycannes en tant qu'agent favorisant la synthèse de macromolécules dermiques et/ou épidermiques.

**11.** Procédé selon la revendication 10, **caractérisé en ce que** la composition comprend en outre une association de trois actifs constituée d'un agent augmentant la synthèse de collagène en tant qu'agent favorisant la synthèse de macromolécules dermiques et/ou épidermiques, d'un agent augmentant la synthèse des lipides épidermiques et d'un agent favorisant la prolifération des kératinocytes.

**12.** Procédé selon la revendication 11, **caractérisé en ce que** la composition comprend en outre au moins un agent stimulant le métabolisme cellulaire et/ou au moins un agent favorisant la microcirculation.

**13.** Procédé selon l'une quelconque des revendications 10 à 12, **caractérisé en ce que** :

- l'extrait de bactérie filamenteuse non photosynthétique non fructifiante est un extrait de *Vitreoscilla filiformis ;*
- l'agent augmentant la synthèse des glycosaminoglycanes est le C-β-D-xylopyranoside-2-hydroxy-propane ou un de ses sels ou isomères optiques et géométriques ;
- l'agent favorisant la synthèse collagène est une protéine hydrolysée de soja ;
- l'agent favorisant la synthèse des macromolécules épidermiques est un extrait de bourgeons de hêtre Fagus sylvatica ;
- l'agent augmentant la prolifération des kératinocytes est le phloroglucinol ou l'un de ses dérivés ;
- l'agent favorisant la synthèse des lipides épidermiques est l'acide cinnamique ou l'un de ses dérivés ;
- l'agent stimulant le métabolisme cellulaire est un extrait de *Saccharomyces cerevisiae ;*
- l'agent favorisant la microcirculation cutanée est la caféine.

**14.** Procédé selon l'une quelconque des revendications précédentes, **caractérisé en ce que** la composition comprend en outre au moins un filtre UVA et/ou UVB.

**15.** Composition cosmétique susceptible d'être utilisée dans le procédé selon l'une quelconque des revendications précédentes renfermant, dans un milieu physiologiquement acceptable, (i) au moins un extrait de bactérie filamenteuse non photosynthétique non fructifiante, (ii) au moins un dérivé C-glycoside.

**16.** Composition selon la revendication précédente, dans laquelle le dérivé C-glycoside répond à la formule générale (I) suivante :

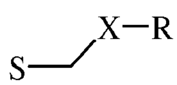

(I)

dans laquelle :

- R représente :

  - un radical alkyle linéaire, saturé en $C_1$ à $C_{20}$, en particulier en $C_1$ à $C_{10}$, insaturé en $C_2$ à $C_{20}$, en particulier en $C_2$ à $C_{10}$, ou un radical alkyle ramifié ou cyclique, saturé ou insaturé, en $C_3$ à $C_{20}$, en particulier en $C_3$ à $C_{10;}$
  - un radical hydrofluoro- ou perfluoro-alkyle, linéaire saturé en $C_1$ à $C_{20}$, en particulier en $C_1$ à $C_{10}$ , ou insaturé en $C_2$ à $C_{20}$, en particulier en $C_2$ à $C_{10}$, ou ramifié ou cyclique, saturé ou insaturé, en $C_3$ à $C_{20}$,

en particulier en $C_3$ à $C_{10}$;
la chaîne hydrocarbonée constituant lesdits radicaux pouvant, le cas échéant, être interrompue par 1, 2, 3 ou plus d'hétéroatomes choisis parmi :

- un oxygène,
- un soufre,
- un azote, et
- un silicium,

et pouvant être éventuellement substituée par au moins un radical choisi parmi :

- $-OR_4$,
- $-SR_4$,
- $-NR_4R_5$,
- $-COOR_4$,
- $-CONHR_4$,
- -CN,
- un atome d'halogène,
- un radical hydrofluoro- ou perfluoro-alkyle, en $C_1$ à $C_6$,
- un radical cycloalkyle en $C_3$ à $C_8$, et/ou

avec $R_4$ et $R_5$ pouvant représenter, indépendamment l'un de l'autre, un atome d'hydrogène, ou un radical alkyle, perfluoroalkyle ou hydrofluoroalkyle linéaire, saturé en $C_1$ à $C_{30}$, notamment en $C_1$ à $C_{12}$, ou insaturé en $C_2$ à $C_{30}$, notamment en $C_2$ à $C_{12}$, ou ramifié ou cyclique, saturé ou insaturé, en $C_3$ à $C_{30}$, notamment en $C_3$ à $C_{12}$ ; ou un radical aryle en $C_6$ à $C_{10}$,
- X représente un radical choisi parmi les groupements :

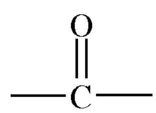

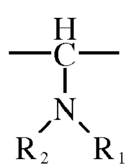

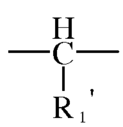

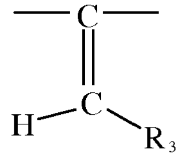

avec $R_1$, $R_2$ et $R_3$ représentant, indépendamment l'un de l'autre, un atome d'hydrogène, ou un radical R, avec R tel que défini précédemment, et $R'_1$ représentant un atome d'hydrogène, un groupe -OH ou un

radical R tel que défini précédemment, $R_1$ pouvant désigner également un radical aryle en $C_6$ à $C_{10}$,
- S représente un monosaccharide ou un polysaccharide comportant jusqu'à 20 unités sucre, en particulier jusqu'à 6 unités sucre, sous forme pyranose et/ou furanose et de série L et/ou D, ledit mono- ou polysaccharide pouvant être substitué par un groupement hydroxyle obligatoirement libre, et éventuellement une ou plusieurs fonction(s) amine(s) éventuellement protégée(s), et

- la liaison S-$CH_2$-X représente une liaison de nature C-anomérique, qui peut être α ou β,

ainsi que leurs sels cosmétiquement acceptables, leurs solvates tels que les hydrates et leurs isomères.

**17.** Composition selon la revendication précédente, dans laquelle S représente un monosaccharide choisi parmi le D-glucose, le D-xylose, le L-fucose, le D-galactose, le D-maltose et notamment le D-xylose.

**18.** Composition selon la revendication précédente, dans laquelle X représente un groupement choisi parmi -CO-, -CH(OH)-, -CH($NH_2$)-, et préférentiellement un groupement -CH(OH)-.

**19.** Composition selon la revendication précédente, dans laquelle R désigne un radical linéaire en $C_1$-$C_4$, notamment $C_1$-$C_3$, éventuellement substitué par -OH, - COOH ou -COOR"$_2$, R"$_2$ étant un radical alkyle saturé en $C_1$-$C_4$, notamment éthyle.

**20.** Composition selon l'une des revendications 15 à 19, **caractérisée en ce que** le dérivé C-glycoside est choisi parmi le C-β-D-xylopyranoside-2-hydroxy-propane et le C-α-D-xylopyranoside-2-hydroxy-propane, et est plus particulièrement le C-β-D-xylopyranoside-2-hydroxy-propane.

**21.** Composition cosmétique susceptible d'être utilisée dans le procédé selon l'une quelconque des revendications 1 à 14, renfermant, dans un milieu physiologiquement acceptable, (i) au moins un extrait de bactérie filamenteuse non photosynthétique non fructifiante, (ii) au moins un agent favorisant la synthèse des glycosaminoglycannes et (iii) au moins une association de trois actifs constituée d'un agent augmentant la synthèse de collagène, d'un agent augmentant la synthèse des lipides épidermiques et d'un agent favorisant la prolifération des kératinocytes.

**22.** Composition selon l'une des revendications 15 à 21, **caractérisée en ce que** les agents sont tels que définis dans la revendication 13.

**23.** Utilisation cosmétique d'au moins un extrait de bactérie filamenteuse non photosynthétique non fructifiante dans une composition, pour diminuer la perte de densité cutanée et/ou la fonte de la lipo-structure de la peau et/ou éviter l'affaissement et/ou le creusement des volumes du visage.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- EP 0604631 A
- EP 0761204 A
- EP 0681831 A
- EP 1354593 A
- FR 9900405
- WO 9402158 A
- WO 9924009 A
- WO 02051828 A


### Littérature non-brevet citée dans la description


- Bergey's Manual of Systematic Bacteriology. 1989, vol. 3